# EUROPEAN PATENT APPLICATION

(11) **EP 0 867 188 A1**
(43) Date of publication of application: **30.09.1998**
(21) Application number: 97935815.7
(22) Date of filing: 19.08.1997
(51) Int. Cl.: A61K 39/00, A61K 9/52

(54) **MICROSPHERES CONTAINING IMMUNOGENS, PROCESS FOR PRODUCING THE SAME, AND METHOD FOR IMMUNIZING ANIMALS WITH THE USE OF THE SAME**

(30) Priority: 20.08.1996 JP 218661/96
(71) Applicant: Freund Industrial Co., Ltd., Tokyo 169 (JP)
(72) Inventor: SUZUKI, Toshiyuki, Freund Industrial Co., Ltd., Shinjuku-ku, Tokyo 169 (JP); YASUMI, Hirotsune, Freund Industrial Co., Ltd., Shinjuku-ku, Tokyo 169 (JP); KUSANAGI, Koichi, Nisseiken Co., Ltd., Ome-shi, Tokyo 198 (JP); HOSHI, Sumio, Ome-shi, Tokyo 198 (JP); IHARA, Takeshi, Ome-shi, Tokyo 198 (JP)
(74) Representative: VOSSIUS & PARTNER
(86) International application number: JP9702864
(87) International publication number: WO9807443

(57) **Abstract**

An immunogen-containing mini-capsule which contains an immunogen for immunizing humans or animals, having a plural-layer structure comprising an immunogen-containing core layer, and shell layers for coating the core layer, the outermost shell layer at least being composed of an intestine-soluble material having shape-retaining ability for retaining the spherical structure at room temperature, and which is useful as a safe and stable oral vaccine for humans and animals.

## Description

### Technical Field:

The present invention relates to a mini-capsule containing an immunogen as an oral vaccine applicable safely and stably to humans and animals, to a process for producing the mini-capsule, and to a method for immunizing animals.

### Background Technique:

For prevention of communicable diseases of humans and animals, an immunogen (vaccine) are widely administered to the humans or animals to develop, in the body, antibodies or cytotoxic T cells specific for the pathogen of the communicable disease.

The vaccine is usually administered by injection to the animals. Disadvantageously, however, the administration operation is not simple, and the inoculum is liable to leave residue at the injection site, or a side effect such as fever, flare, amyotrophy, and shock is liable to be caused.

In the case where the treated objects are animals, especially industrial animals such as bovines, swine, and fowls, the labor of injection is enormous for immunizing a large number of animals in a short time. In the case where the treated objects are fared fish, administration by injection cannot readily be practiced. Therefore, a simple oral administration method is demanded for objective animals in many cases.

Regarding immune response, it is known that numerous immunocompetent cells exist in intestinal mucosa and constitute mucosal immune system to provide a primary defense mechanism against various pathogens invading through the mucosa. The B-lymphocytes are considered to have homing routes common to mucosae to serve to make the immunity developed in the intestine to be effective in the mucosae in the whole body.

Thus, the immunization by stimulating the intestinal lynphoid tissues could be effective against various pathogens. Therefore, administration of oral vaccine, which is simple and natural, has generally been considered to be suitable for delivering an immunogen to intestine for immunizing a living body.

In spite of the above advantages, few oral vaccines have been effectively used practically.

One reason for this is that the degradation of immunogen thereof occurs before the immunogen reaches the intestine. In other words, the immunogen can be inactivated by action of the acid and the protein-decomposing enzyme (protease) in the stomach during the delivery to the intestinal mucosa following oral administration.

The known oral administration type liquid vaccines include an acute poliomyelitis vaccine and a Newcastle disease vaccine - The former has been practically used for several tens of years. The liquid vaccines are practicalized owing to its inherent resistance to the acid and the protease in the stomach or owing to the immuno-stimulating site located before the stomach where the immunogen will be degraded. Therefore, this method cannot be applied universally, and the pathogens (immunogens) which are applicable as the oral vaccine are strictly limited.

One effective method to overcone the above problems can be administration of the immunogen enclosed in a capsule (intestine-soluble capsule) which is not digested in the stomach but is soluble in the intestine.

However, the immunogen which is mainly consisted of protein is liable to be inactivated by physical factors such as temperature, moisture, light, and shaking. No constitution or no production method has been disclosed which is suitable for the intestine-soluble capsule.

For example, JP-B-53-5371 regarding a vaccine having an intestine-soluble coating layer discloses a method for protecting swine against transmissible gastroenteritis virus by oral administration of a fine particulate attenuated vaccine coated with an intestine-soluble coating layer which is hardly soluble in a stomach but is soluble rapidly in a small intestine and in a shape of a sphere of a size less than about 2 mm for passing through the pyloric valves to the small intestine before destruction of the vaccine by gastric juice attack. The above patent publication discloses specifically a pellet having an intestine-soluble coating layer (cellulose acetate phthalate : CAP) as the administration form. However, the details of the "pellets" described in the patent publication is not clear, and the method of coating of the pellet with CAP is not described. Assuming the above coating to be conducted by an usual method, a solution of CAP in an organic solvent is sprayed in a coating chamber and the coated material is dried by a hot air stream. In this method, the vaccine will be inactivated by the heating and the organic solvent. Further, the CAP coating film disclosed in the above patent publication can be dissolved in the stomach within 2 hours, so that the pellets are made smaller than 2.0 mm for passing through the stomach in a short time to avoid the dissolution of the coating. From these descriptions, the pellet disclosed in the patent publication does not give a satisfactory intestine-soluble vaccine.

JP-A-3-173829 discloses a stable intestine-soluble immunization composition for oral administration in a dry sphere shape. This composition comprises an immunogen for immunizing humans or animals and gelatin having an average molecular weight ranging from 80,000 to 120,000 and a jelly strength of not less than 150. This patent publication also discloses a process for producing the composition in which gelatin and a vaccine are mixed uniformly in physiological saline at a low temperature, the mixture is heated to become a sol and is poured into a hydrophobic liquid such as liquid paraffin to form single spheres, and the spheres are dried at a low temperature to form an intestine-soluble coating layer. This method, however, is described to form the intestine-soluble coating film by a flow coater (fluidized layer coating apparatus) or a centrifugal granulator, so that the method involves the similar disadvantages as the process of the above JP-B-53-5371, not giving satisfactory intestine-soluble vaccine. Furthermore, in the above patent laid-open publication, the stability of the vaccine is not confirmed for longer than six weeks.

JP-A-62-195324 discloses coating of a soft gelatine capsule with a polyacrylic polymer as the intestine-soluble coating material for the purpose of DDS (drag delivery system). The coating of the polyacrylic polymer involves the problems similar to the above method.

JP-A-55-86463, JP-A-55-146160, JP-A-57-154119, and so forth disclose intestine-soluble hard capsules, which are not suitable for production of fine capsules for administration to animals.

JP-A-7-145082 discloses an intestine-soluble capsule formed by crosslinking of 100 parts of gelatin and 1 to 40 parts of carrageenan. The disclosure mentions only the production of seamless capsules of 1 mm particle in diameter by a double-nozzle method, and not the production of a vaccine.

In view of the above prior art techniques, the present invention intends to provide an immunogen-containing mini-capsule useful for a safe and stable oral vaccine applicable to humans and animals, a process for production thereof, and a method for immunization of animals.

The inventors of the present invention considered that only few practical vaccines are provided because the techniques of the above disclosure of intestine-soluble capsules to deliver antigens to small intestine are lacking for producing a practical carrier capable of delivering the immunogen without inactivation of the above disclosure of intestine-soluble capsules to deliver antigens to small intestine. Specifically the problems thereof include inactivation of encapsulated immunogen by the partial dissolution of capsules in the stomach owing the insufficiency of enteric coating or by the organic solvent and the heat in the process of the production of capsules, complexity of the production process resulting in a long production time and a high production cost to be not suitable for commercial production; inappropriate size of capsules for passing through the stomach to cause the inactivation of immunogen owing to long retention time in the stomach and penetration of the gastric juice into the particle to deactivate the immunogen; and insufficient storability of the immunogen of the intestine-soluble vaccine in a capsule to cause extremely rapid inactivation of the immunogen for market distribution. The present invention intends to provide a practicable intestine-soluble capsule by solving the above problems.

An object of the present invention is to provide a novel intestine-soluble capsule for enclosure of an immunogen. This type of capsule has the characteristics as follows: the capsule having a form for passing through a stomach in a suitable residence time, being produced in a simple process, preventing inactivation of the immunogen sufficiently, and being capable of storing the enclosed immunogen stably for a usually required period for commercial distribution.

Another object of the present invention is to provide an intestine-soluble capsule for easy administration to a living body, and being advantageous in administration to animals such as administration by mixture with bait, while realizing the aforementioned object.

### Disclosure of the Invention:

The characteristics of the present invention for achieving the above objects are set forth in the respective items of the scope of the above patent claims.

The immunogen-containing mini-capsule of claim 1 of the present invention is a mini-capsule (hereinafter occasionally referred to as a "capsule") having a plural layer structure and containing an immunogen for immunizing humans or animals, and characterized in that the mini-capsule has a plural layer structure comprising a spherical core layer and one or more spherical shell layer, and at least the outermost shell layer is constituted of an intestine-soluble material capable of retaining its spherical shell structure at room temperature. The room temperature means a temperature environment generally ranging from 20°C to 25°C, and excludes exceptional conditions such as refrigeration.

The mini-capsule of the present invention has a size preferably ranging from 0.2 to 8 mm, more preferably from 0.5 to 5 mm. The mini-capsule having a size larger than 8 mm tends to be unsuitable for oral administration and is liable to cause inactivation of the vaccine by residing for a longer time in a stomach, whereas the mini-capsule having a size of less than 0.2 mm is not sufficient in securing the resistance to the gastric juice because of a larger surface area relative to the volume. Therefore, the above size range is preferred.

The mini-capsule of the present invention should have at least an outermost shell layer constituted of an intestine-soluble material, and should have the aforementioned shape-retaining property. The "shape-retaining property" herein means a property of retaining the spherical shape of the mini-capsule, and the outermost layer is required to have this property. In prior art techniques, the capsule is constituted of a spherical material having a shape-retaining property and an intestine-soluble coating layer applied thereon having no shape-retaining property by itself. Therefore, in the process of the coating with the intestine-soluble material, the immunogen can be adversely affected by heat or by the organic solvent to be inactivated, which should be avoided. In a certain coating process, complete intestine-solubility cannot be achieved as shown in JP-B-56-5371. Accordingly in this invention, the intestine-soluble material layer is essentially made thick to secure sufficiently the resistance to gastric juice and the shape-retaining property.

The mini-capsule of the present invention has a plural layer structure having an intestine-soluble outermost capsule shell layer and an immunogen-containing substance enclosed therein. The immunogen-containing substance includes dispersions of the immunogen in an aqueous medium or an oily medium, dispersions thereof in an oily paste or a solid, dispersions thereof in a water-soluble solid, and dispersions thereof in an intestine-soluble solid. The immunogen may be separated by an oily interlayer from the outer shell layer of the capsule. The immunogen-containing layer may contain an additive such as a stabilizer. In the above constitution, the interlayer is preferably non-fluid at room temperature or the storage temperature in view of the shape-retaining property and the strength of the layer, and prevention of occurrence of uneven layer thickness in the production process. The stabilizer is added to keep the immunogenicity of the immunogen: for example, sugar and protein for a pathogen to keep the infectivity.

The mini-capsule of the present invention having the aforementioned constitution can be produced by forming a spherical particle of a plural layer structure, which is solid at room temperature, by employing a core liquid comprising an immunogen suspended in a liquid (for example, aqueous solution) not containing an organic solvent which may inactivate the immunogen; an outermost shell layer liquid comprising an intestine-soluble material not containing an organic solvent which may inactivate the immunogen; and optionally, an interlayer liquid not containing an organic solvent which may inactivate the immunogen. The materials and the process for producing the respective layers should be selected such that each of the layers can be formed for spherical coating with sufficient fluidity at a temperature not affecting adversely thermally to inactivate directly or indirectly the immunogen. The contact temperature of the medium (preferably an aqueous suspension of gelatin or the like) with the immunogen for dispersion is preferably not higher than 50°C, more preferably not higher than 40°C, and the temperature for outer layer formation for coating the immunogen-containing core layer is preferably not higher than 90°C, more preferably not higher than 70°C in most cases.

As described above, the intestine-soluble material necessary for constituting the mini-capsule of the present invention is suitably constituted mainly of gelatin in view of the production process. A natural rubber such as gellan gum, carrageenan, xanthane rubber, and gum arabic is preferably added thereto for resistance to gastric juice. A polyvalent metal ion such as calcium, magnesium, and aluminum may be added further thereto. Thereby, sufficient intestinal-solubility can be obtained. To the gelatin, a plasticizer such as glycerin, propylene glycol, and sorbitol may be added, glycerin being particularly suitable.

The mini-capsule of the present invention has the outermost shell layer made preferably of a gelatin-based mixture, and the enclosed material (core layer) composed preferably of a dispersion of the immunogen in gelatin or a gelatin-based intestine-soluble material and solidified by cooling.

The mini-capsule of a triple structure may have a center portion (core layer) composed of a solid of powder or gelatin containing an immunogen, and an interlayer composed of an oily or lipophilic substance having a flow temperature of not lower than 10°C, preferably not lower than 15°C to obtain a constitution in which the immunogen is less susceptible to the moisture of the outermost shell and the environment. The oily or lipophilic substance includes mixtures of lecithin and glycerides, especially medium-chain fatty acid triglyceride (MCT); sucrose fatty acid esters; and mixtures of a sucrose fatty acid ester and MCT. The preferred mixing ratio of the lecithin to the medium-chain fatty acid triglyceride ranges from 1:9 to 7:3.

The triple-structured mini-capsule in which the immunogen-containing material is powdery can be produced by dropping an immunogen containing aqueous solution and removing the aqueous component through a drying process to form a powdery material. Although the mini-capsule decreases its volume with the removal of the water from the core liquid, the capsule shape can be retained by the shape-retaining property of the outer shell layer.

The constitution material for the layer for constituting the mini-capsule of the present invention is preferably to be selected such that the flow temperature of the outermost shell is not lower than 20°C, more preferably not lower than 25°C to secure the shape-retaining property of the mini-capsule during storage and use.

The aforementioned mini-capsule of the present invention is preferably produced by discharging plural liquids through a concentric multiple nozzle with an aqueous intestine-soluble material solution as the outermost shell layer into a solidifying bath for solidification. In the case where the concentric multiple nozzle is a concentric triple nozzle, the aqueous immunogen suspension may be extruded through the center tube of the concentric triple nozzle; a hydrophobic material through the intermediate nozzle, and the aqueous intestine-soluble material solution through the outermost nozzle. The solidification liquid includes liquid paraffin, vegetable oils such as colza oil, and MCT and silicone oil.

The capsule containing an immunogen is applicable to domestic animals such as bovines, equines, swine, ovines, caprines, minks, and fowls; pet animals such as canines, felines, and aquarium fishes; experimental animals such as mice, rats, guinea pigs, hamsters, lagomorphs, and ferrets; farmed fishes such as young yellowtails, ayu fishes, eels, sea breams, flatfishes, carps, rainbow trouts, and yellowtails; and wild animals such as raccoon dogs, and foxes. The immunogen includes inactivated vaccines, attenuated vaccines, detoxified vaccines, and component vaccines (including peptide vaccines). Also, gene recombinant vaccines, and DNA vaccines in another classification are effective.

The vaccine of the present invention consisting of an attenuated strain capable of replicating in the intestine as the immunogen is particularly effective, since the attenuated vaccine strain can replicate in the intestine to stimulate the immune system. In use of an attenuated living virus as the immunogen, it is especially important to prevent from inactivation throughout the production process and the application steps (capsule formation, storage before use of the capsule, and passage through a stomach). The conditions proposed by the present invention avoiding the exposure of the pathogen to a high temperature, an organic solvent, high humidity for a long time, and an extreme ph are basic universal conditions for prevention of the inactivation, although the necessary specific conditions depend on the respective pathogens. The process of the present invention using no organic solvent is highly effective in a virus or a bacteria having a lipid membrane (e.g., enveloped virus, including the TGE virus mentioned later), since the virus or the bacteria is extremely susceptible to the organic solvent capable of dissolving the membrane.

The present invention is applicable for any immunogen for immunizing humans and animals for prevention of infectious diseases.

For example, the immunogen includes inactivated vaccines against influenza, Japanese encephalitis, pertussis, cholera, pneumococcus, Wyle's disease, dysentery, typhoid, paratyphoide, epidemic typhus, scarlatine, epidemic cerebrospinal meningitis, pest, Lassa fever, malaria, yellow fever, anthrax, infectious diarrhea, trombiculiasis, filariasis disease, relapsing fever, schistosomiasis, trachoma, syphilis, gonorrhea, chancroid, lymphogranuloma inguinalis, leprosy, Newcastle disease, infectious coryza, avian infectious bronchitis, infectious bursal disease, mycoplasma gallisepticum infection, fowl cholera, fowl plague, pullorum disease, duck plague, viral hemorrhagic disease of rabbits, swine infections atrophic rhinitis disease, swine actinobacillosis, porcine epidemic diarrhea, porcine rotavirus infection, Aujeszky's disease, transmissible gastroenteritis of swine, porcine adenovirus infection, porcine reproductive and respiratory syndrome (PRRS), porcine parbovirus infection, Glässer's disease, African swine fever, swine vesicular disease, vesicular stomatitis, swine dysentery, equine influenza, equine rhinopneumonitis, equine getah virus infection, equine viral arteritis, glanders, equine infectious anemia, pseudofarcy, equine paratyphoid, African horse sickness, equine encephalomyelitis, foot and mouth disease, rinderpest, bovine ephemeral fever, Ibaraki disease, Chuzan disease, Akabane disease, infectious bovine rhinotracheitis, bovine viral diarrhea, parainfluenza virus infection in cattle, bovine adenovirus infection, bovine rotavirus infection, bovine contagious pleuropneumonia, blackleg, hemorrhagic septicemia in cattle, brucellosis, Johne's disease, piroplasmosis, anaplasmosis, bluetongue, Rift Valley fever, malignant catarrhal fever, heart water, sheep pox, maedi-visna, contagious caprine pleuropneumonia, feline calicivirus infection, feline viral rhinotracheitis, feline panleukopenia, rabies, distemper, canine infectious hepatitis, canine infectious laryngotracheitis, canine parainfluenza, canine parvovirus infection, canine coronavirus infection, canine herpesvirus infection, canine oral papillomatosis, feline infectious peritonitis, feline leukemia, vibriosis in fish, furunculosis, and the like; attenuated vaccines against acute poliomyelitis, measles, rubella, tuberculosis (BCG), typhoid, variola, epidemic parotitis encephalitis, Newcastle disease, avian infectious laryngotracheitis, avian infectious bronchitis, fowl pox, infectious bursal disease, Marek's disease, avian encephalomyelitis, porcine epidemic diarrhea, rotavirus infection, Aujeszky's disease, swine fever, swine erysipelas, transmissible gastroenteritis of swine, Japanese encephalitis, rinderpest, Ibaraki disease, Akabane disease, infectious bovine rhinotracheitis, bovine viral diarrhea, parainfluenza virus infection in cattle, bovine adenovirus infection, feline calicivirus infection, feline viral rhinotracheitis, feline panleukopenia, and the like; detoxified vaccines against diphtheria, tetanus, habu toxoid, typhoid, botulism, and the like; and component vaccines against influenza HA, hepatitis B, hepatitis C, herpes, and the like.

The immunogen-containing mini-capsule of the present invention can be administered as an oral vaccine to a human or an animal, and can be given naturally to a domestic animal by mixing with a dried bait. Moreover, several kinds of capsules may be mixed with the bait to confer the immunity against several pathogens to the animal simultaneously.

### Brief Description of the Drawings:

Fig. 1 shows schematically constitution of an apparatus for producing the immunogen-containing mini-capsules of the present invention.

Fig. 2 shows a state of production of immunogen-containing mini-capsules of Type I (double structure): (a) showing the state of extrusion of mini-capsules through a nozzle, and (b) showing the structure of the produced double-layered immunogen-containing mini-capsule.

Fig. 3 shows a state of production of immunogen-containing mini-capsules of Type II (triple structure): (a) showing the state of extrusion of mini-capsules through a nozzle, and (b) showing the structure of the produced triple-layered immunogen-containing mini-capsule.

### Best Mode of Practicing the Invention:

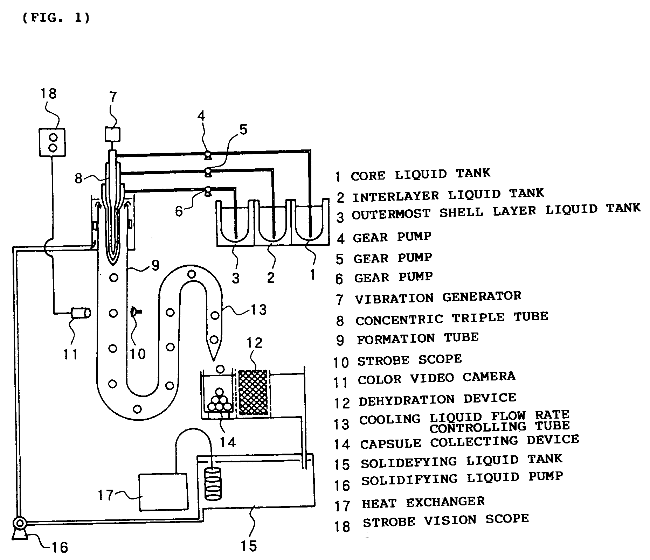

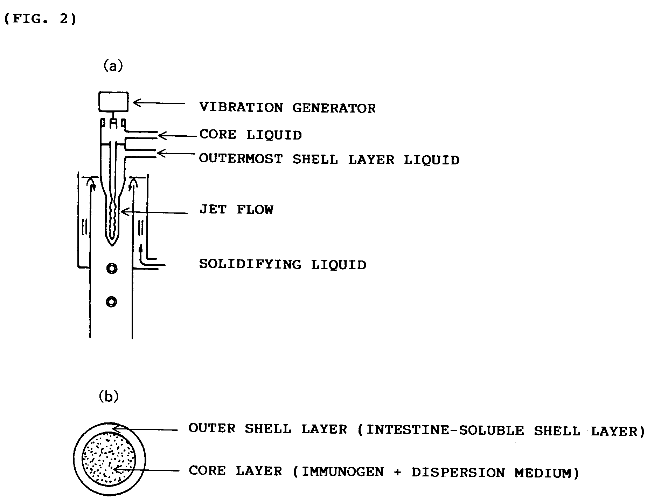

Fig. 1 shows an example of schematic construction of an apparatus for producing the mini-capsules. Two types of structures of mini-capsules can be produced: (1) the structure constituted of a core layer containing an immunogen dispersed in a solid dispersion medium, and an outer intestine-soluble shell layer (Type I); (2) the structure having an immunogen at the center as a core layer and a lipophilic interlayer for separation from an intestine-soluble outermost shell layer (Type II). The two types of capsules can be produced respectively by use of a double nozzle or a triple nozzle. In Fig. 1, the nozzle parts are shown as a triple nozzle. Therefore, Type I capsule can be produced by an apparatus of a simpler structure in the nozzle shape and the source material system. Regardless of the types, the basic structure is similar. Fig. 2 shows the part of the shape of the nozzle.

The process is described for producing the mini-capsule of the present invention by means of the apparatus shown in Fig. 1. When a double nozzle is employed, the devices denoted by the numerals 2 and 5 are not necessary.

The mini-capsules are produced as below.
(1) A core liquid containing an immunogen is delivered from a tank 1 by a pump 4 to the inner tube of a nozzle 8 (concentric triple nozzle).
(2) An interlayer material solution is delivered from a tank 2 by a pump 5 to the intermediate tube of the nozzle 8 (in the case of production of Type II).
(3) An outermost layer material solution is delivered from a tank 3 by a pump 6 to the outer tube of the nozzle 8.
(4) The respective solutions sent to the nozzle 8 are allowed to flow down (to drop) from solution-discharging outlets into a solidifying liquid to form a "jet" in a solidification tube 9. The nozzle 8 is vibrated by a vibration generator 7 to form uniform liquid droplets.
   In the formation tube 9, solidifying liquid is continuously fed from the upside by overflow by means of a pump 16, and the solution discharging outlets of nozzle 8 are immersed in the solidifying liquid.
(5) The "jet" forms, owing to "surface tension", spheres in which the core liquid is enclosed by the outermost shell layer solution (an interlayer material solution is present for Type II).
(6) The formed droplets are in a uniform drop shape in correspondence with the vibration frequency of the vibration generator 7 (generally about 10 to about 300 Hz). The droplet shape can be observed clearly by a strobo-vision scope 18 and a color video camera 11, and the vibration frequency may be controlled thereby, if necessary.
(7) The droplets are transported by the flow of the solidifying liquid and are solidified completely during the transportation and are delivered to a cooling liquid volume-adjusting tube 13.
(8) The droplets delivered to the cooling liquid volume-adjusting tube 13 are then allowed to drop into a capsule-collecting device 14 to be separated from the solidifying liquid.
(9) The cooling liquid volume-adjusting tube 13 in this embodiment is provided such that the flow rate of the solidifying liquid can be adjusted by changing the vertical position thereof.
(10) The solidifying liquid separated from the droplets is treated by a dehydration device 12 to remove the water, and then sent to a solidifying liquid tank 15, and recycled after cooling to a prescribed temperature by a heat-exchanger 17 by a solidifying liquid pump 16 to the formation tube 9.

### Example 1

### (Production of Mini-capsules)

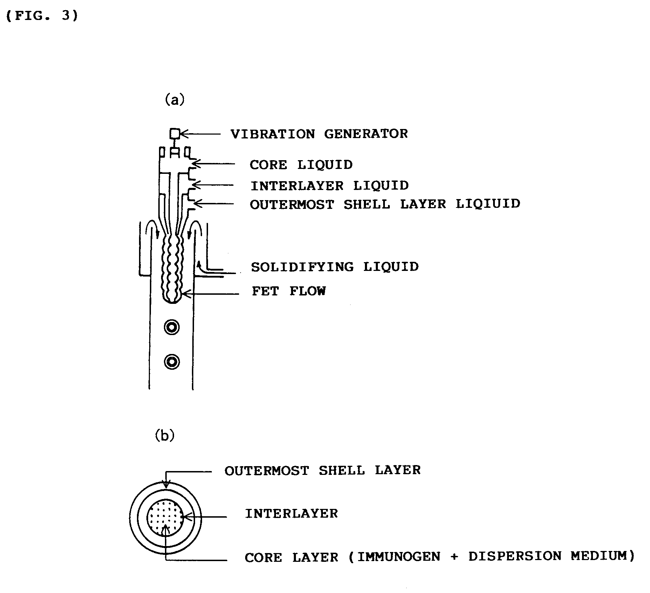

The mini-capsules of the above Type I (shown in Fig. 2), and of the above Type II (shown in Fig. 3) were produced respectively with the above-described apparatus. Table 1 shows the formulations of the respective liquids and the production conditions.

### (Table 1)

In Table 1, "h-5 strain" is an attenuated virus derived from a virus (having a lipid membrane) of transmissible gastroenteritis of swine (TGE), and is in a state of an aqueous liquid containing a stabilizer (10% lactose) and other additives. This h-5 strain, attenuated virus, is protease-sensitive, and its infection site is dislocated to the respiratory tract. This virus will be inactivated normally under environments of stomach, and cannot replicate in a small intestine. The h-5 strain is selected as a suitable material for the test of the effect of the intestine-soluble preparation. Naturally the present invention is not limited thereto. The virulent TGE virus infects the small intestine of infant swine to cause severe aqueous diarrhea to kill most of the swine of seven day age or younger.

The production under the conditions of Table 1 proceeded normally. The product was dried by an air-flow dryer with stirring at 15°C for 10 hours to obtain mini-capsules. Table 2 shows the properties of the obtained mini-capsules.

### (Table 2)

### (Properties of Mini-capsules in Immunity)

### (1) Intestinal Solubility Test of Mini-capsules

The capsules corresponding to 1 ml of the virus suspension in terms of the volume before preparation of the mini-capsules were weighed out (Type I: 0.38 g, Type II: 0.71 g). The capsules were allowed to suspend in 10 ml of an artificial gastric juice (pepsin 0.32%, NaCl 0.2%, ph being adjusted to 1.3-1.6 with hydrochloric acid). The suspension was incubated by immersion into water bath at 37°C for 90 minutes with gentle shaking at intervals of about 10 minutes. The capsules were removed from the artificial gastric juice, and were washed once with distilled water (ph 5.0). After removal of the distilled water, the mini-capsules were dissolved by adding 10 ml of PBS (ph 6.8) and shaking at 37°C for 15 to 30 minutes. This virus suspension was regarded as ten-fold diluted, and measured for the virus infectious dose using swine testicle-derived cells (ST cells)

For the virus titration, ST cells were cultured in a monolayer on a 48-well plate. After removal of the culture medium, the test samples of sequential 10-fold dilution were inoculated respectively into 4 wells in an amount of 0.1 ml per well. 0.5 ml of a culture medium for cell maintenance was added to each of the wells. The culture was incubated in a carbon-dioxide incubator at 37°C for one week and development of the cytopathic effect (CPE) was observed. The quantity of the infectious virus was calculated by the Karber method and was represented by log₁₀TCID₅₀/ml (50% for tissue culture infectious dose). (The quantity of the virus is shown hereafter in the same manner.)

### (Table 3)

Table 3 shows that the rate of inactivation of the encapsuled virus was 1/100 to 1/1000 times less than that of the non-mini-capsuled virus, and that any of the mini-capsules protected the TGE virus satisfactorily.

Since the mini-capsules were produced in two lots under the same conditions, the results are shown for Lot Nos. 1 and 2.

It was observed to the naked eye that the shape of all the mini-capsules was retained in the artificial gastric juice and was dissolved in the artificial intestinal juice.

### (2) Virus Stability Test

Respective types of mini-capsules were prepared by aforementioned production process using vaccine, of which virus infectious dose was 9.25 before encapsulation. The mini-capsules were divided into glass vials. The vials were evacuated and tightly sealed, or were evacuated and then purged with nitrogen and tightly sealed, and were stored at -70°C, or 4°C. The change of the virus infectious dose was measured. The virus infectious dose was 8.50 immediately after the mini capsulation.

### (Table 4)

Table 4 shows that the virus was stably stored for 6 months under any of the conditions.

### (3) The Results of Oral Administration Test

The mini-capsule preparation of Type II corresponding to the virus infectious dose of 9.00 was mixed with the bait and administered to two swine (Nos. 86 and 87) by natural feeding. The neutralizing titer in the serum of each of the swine was measured with lapse of time. Table 5 shows the results.

### (Table 5)

Table 5 shows that the orally-administered swines exhibited rise in high antibody titer through 17 weeks.

The mini-capsule preparation of three-layer structure containing TGE virus of infectious dose of 9.00 was administered to a swine in late pregnancy period by natural feeding. The neutralizing antibody titer in the serum and in the milk after the partus were measured with lapse of time. Tables 6 and 7 show the results. The mother swine gave birth on 18th day after administration of the mini-capsules.

### (Table 6)

### (Table 7)

Tables 6 and 7 show that the neutralizing antibody titer in the serum of the mother swine increased, and the neutralizing antibody was detected also in the milk. This indicates that the oral administration of the mini-capsule preparation developed the systemic immune response (induction of the antibody response in blood) as well as local immune response (induction of the antibody response in the milk).

This Example 1 describes the test results in the case of the mini-capsules prepared using gellan gum. Similar results were also obtained in the tests in the case of mini-capsules prepared using combinations of gum arabic with a calcium salt, and carrageenan with a calcium salt (both composed of 9.47 parts of gelatin, 0.40 parts of gum, and 0.13 parts of calcium chloride).

### Example 2 and 3

The mini-capsules were prepared in the same manner as in Example 1 with the formulations and under the conditions shown in Table 8. The production was conducted smoothly. The products were dried under the same conditions as in Example 1. Table 9 shows the properties of the resulting mini-capsules.

### (Table 8)

### (Table 9)

### (Intestinal Solubility of Mini-capsules)

In the tests, artificial gastric juice 1 corresponding to the stomach conditions of a hungry swine (ph 1 to 3) described below and artificial gastric juice 2 corresponding to the stomach conditions after feeding (ph 4 to 6) described below were used as the artificial gastric juice, and the artificial intestine juice described below were used.
Artificial gastric juice 1: 0.2% NaCl, 1% pepsin (1:10000, produced by Nacarai tesque K.K.), ph 1.3 (adjusted by HCl)
Artificial gastric juice 2: 0.2% NaCl, 1% pepsin (as above), ph 5.6 (adjusted by HCl)
Artificial intestine juice: 0.05 M KH₂PO₄, ph 6.8 (adjusted by NaOH)

The tests were conducted using the above artificial gastric juices 1 and 2, and the above artificial intestine juice. Firstly, three samples of 1.23 g of the mini-capsules corresponding to 1 ml of the core liquid (vaccine solution) before encapsulation were weighed out as the test samples. Two samples were washed once with a small amount of artificial gastric juice 1 or 2. To each samples, 10 ml of the respective artificial gastric juice was added, and the mixture was shaken in a water bath at 37°C for 90 minutes. The treated mini-capsule sample was washed twice with the respective artificial gastric juice through a mesh (cell Strainer; manufactured by Falcon Co.). Then 10 ml of the artificial intestine juice was added to the washed mini-capsule sample, and the mixture was shaken in a water bath at 37°C for 60 minutes. The virus infectious dose in the external liquid was measured. For the measurement of virus infectious dose in mini-capsules without any treatment, mini-capsules were crushed with a mortar in a 10 ml of the artificial intestine juice and recovered samples were titrated. Table 10 shows the results.

### (Table 10)

The mini-capsules of the both preparations protected the virus sufficiently from the artificial gastric juice, and the virus was released by the incubation with the artificial intestine juice. Incidentally, the uncapsulated virus was completely inactivated by the incubation with artificial gastric juice 1 for 90 minutes, and the virus infectius dose value of the virus dropped to a 1/1000 level by the incubation with artificial gastric juice 2.

### Industrial Availability

The effects described below are achieved by the present invention.

According to the invention of claims 1 to 5 in which an immunogen for immunizing humans or animals is enclosed in a intestine-soluble material having a shape-retaining property, there is provided an immunogen-containing mini-capsule preparation which is useful for a safe and stable oral vaccine for humans or animals. Since immunogen-containing mini-capsules stimulate systemic and local immune systems to induce, especially, effective mucosal immune response following oral administration, humans or animals can be protected from infections of many pathogens by the prevention of entry at mucosal surfaces.

According to the above invention, there is provided a practically useful oral administration vaccine which achieves effects of causing no pain of humans and animals on injection, saving greatly the labor of inoculation to industrial animals, and eliminating side effects caused by injection such as residual inoculum at the action site, fever, flare, amyotrophy, and shock.

Furthermore, according to the present invention of an immunogen-containing mini-capsule for immunizing humans and animals which has a plural layer structure constituted of an immunogen-containing core layer, and one or more layers for coating the core layer, and the outermost shell layer composed of an intestine-soluble material having shape-retaining ability for retaining the spherical shell structure, there is provided an immunogen-containing mini-capsule preparation for use as a stable and safe oral vaccine for humans and animals. In this immunogen-containing mini-capsule preparation, immunogen is less liable to be inactivated, and is especially effective in the case of an attenuated pathogen, since the core layer containing the immunogen is coated with an intestine-soluble outermost layer prepared from an aqueous solution; This coating method is different from the conventional ones in which the immunogen is brought into contact with an organic solvent and is exposed to a high temperature. The immunogen-containing mini-capsule can be produced in a simple process in high productivity at a low cost advantageously.

Furthermore, the mini-capsule (capsule) of the present invention is capable of retaining the stability of the immunogen to be delivered to the intestine without direct contact of the immunogen with the gastric juice owing to protection by the outermost shell of the intestine-soluble material. The mini-capsule (capsule) of the three-layer structure keeps the stability of the immunogen higher and maintains the preservability for a relatively long period in commercial distribution owing to the oily or lipophilic material intercepting effectively the core layer from external atmosphere (moisture and oxygen).

According to the invention of claims 6 and 7, the aforementioned mini-capsule of a plural-layer structure can be produced effectively with high productivity.

According to the invention of claim 8, labor for administration is saved practically by natural feeding of the mixture, for example, with bait to animals, especially domestic animals.

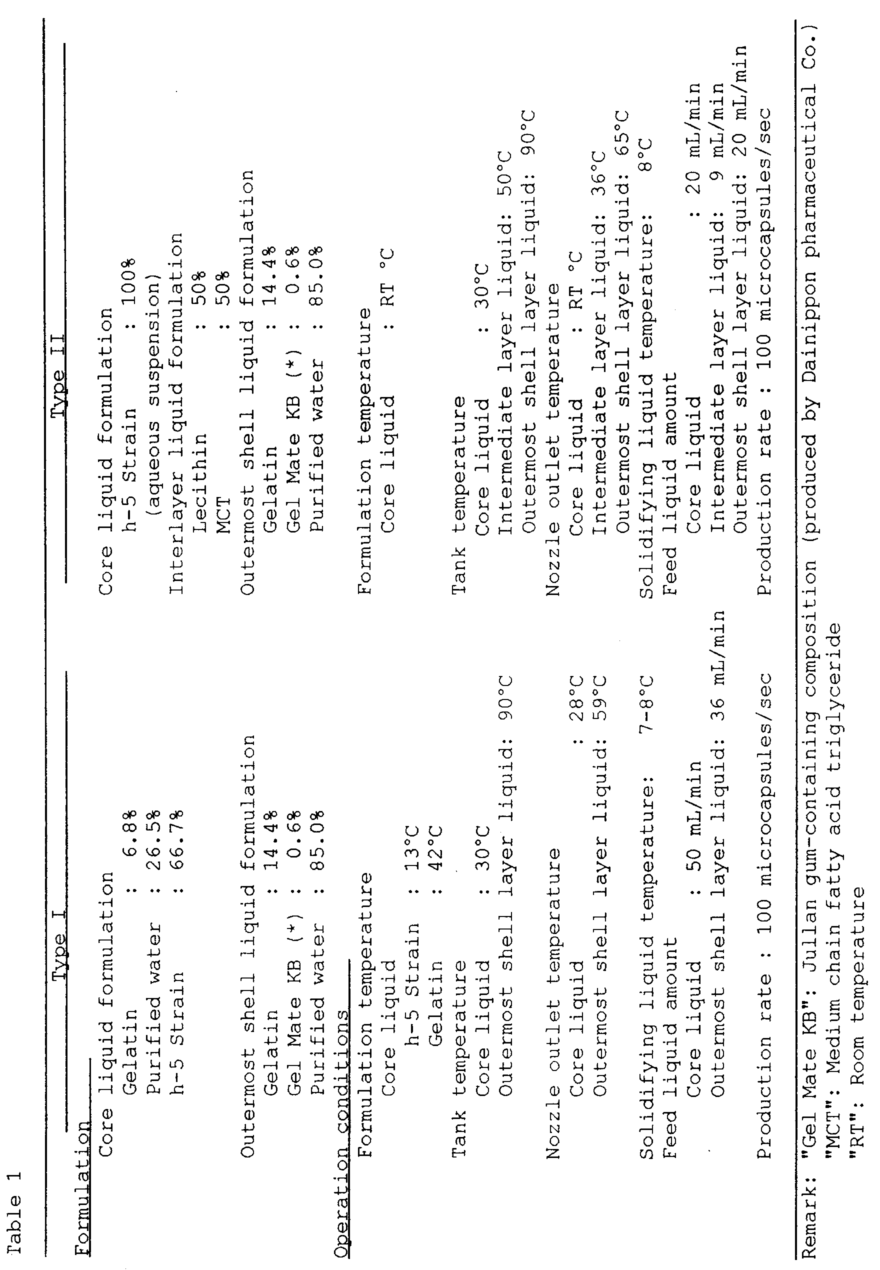

**Table 2**

| Type I | | Type II | |
|---|---|---|---|
| | | | |

| Microcapsules | | | |
|---|---|---|---|
| Before drying | | Before drying | |
| Particle diameter | 3.0 mm⌀ | Particle diameter | 2.5 mm⌀ |
| Weight | 14 mg | Weight | 8.2 mg |

| After drying | | After drying | |
|---|---|---|---|
| Particle diameter | 2 mm⌀ | Particle diameter | 1.6 mm⌀ |
| Weight | 3.9 mg | Weight | 2.3 mg |

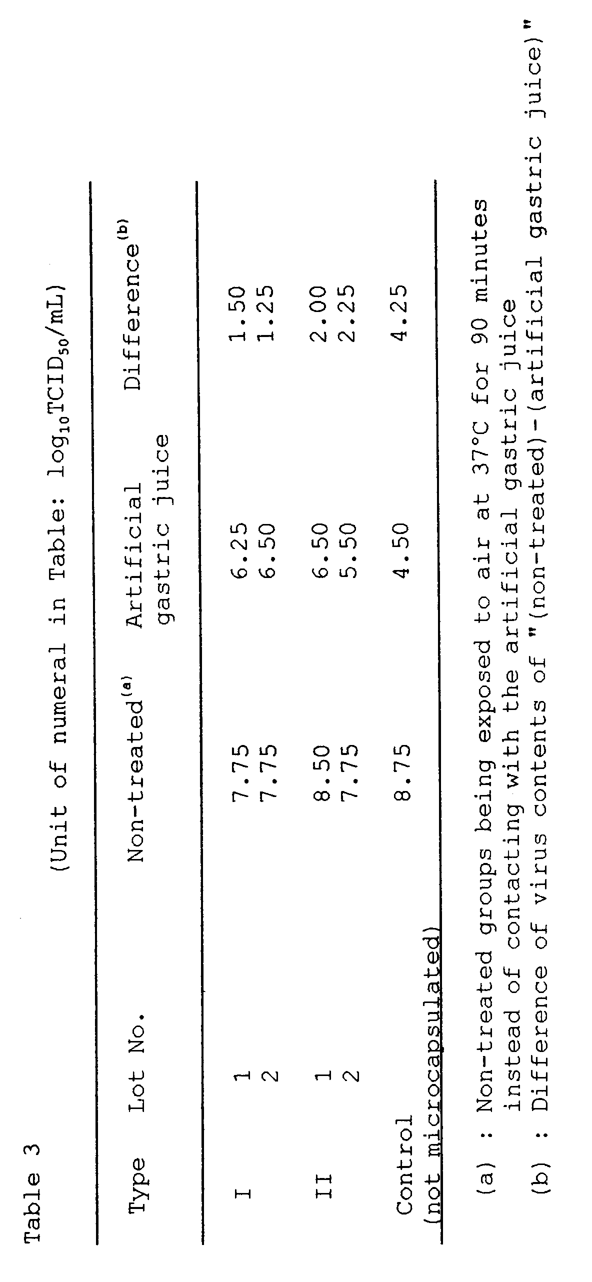

**Table 4**

| Type | Storage conditions | | Virus content (log₁₀TCID₅₀/mL) | |
|---|---|---|---|---|
| | | | 3 months | 6 months |
| I | Vacuum | -70°C | 7.75 | 7.50 |
| | | 4°C | 7.50 | 6.75 |
| | Nitrogen | -70°C | 8.00 | 7.25 |
| | | 4°C | 7.00 | 7.00 |
| II | Vacuum | -70°C | 8.25 | 7.75 |
| | | 4°C | 7.75 | 8.00 |
| | Nitrogen | -70°C | 8.25 | 7.50 |
| | | 4°C | 8.00 | 7.50 |
| Remark: The virus content at the start of storage was 8.50 in every sample. | | | | |

**Table 5**

| Swine No. | Weeks | | | | | | | | | | | | | |
|---|---|---|---|---|---|---|---|---|---|---|---|---|---|---|
| | 0 | 1 | 2 | 3 | 4 | 6 | 8 | 10 | 11 | 12 | 14 | 15 | 16 | 17 |
| 86 | <2 | 64 | 64 | 256 | 256 | 128 | 128 | 128 | 128 | 256 | 128 | 256 | 256 | 256 |
| 87 | <2 | 16 | 32 | 64 | 128 | 64 | 64 | 32 | 64 | 128 | 128 | 128 | 64 | 128 |

**Table 6**

| Neutralizing Antigen Titer of Serum of Pregnant Swine Dosed with TGE Virus-Containing Microcapsule Preparation | | | | | |
|---|---|---|---|---|---|
| Weeks after administration | 0 | 1 | 2 | 3 | 4 |
| Neutralizing antigen titer | <2 | <2 | 8 | 64 | 64 |

**Table 7**

| Neutralizing Antigen Titer of Milk of Pregnant Swine Dosed with TGE Virus-Containing Microcapsule Preparation after Parturition | | | | | | | |
|---|---|---|---|---|---|---|---|
| Days after parturition | 0 | 1 | 2 | 3 | 4 | 6 | 7 |
| Neutralizing antigen titer | 32 | 32 | 16 | 8 | 8 | 4 | 8 |

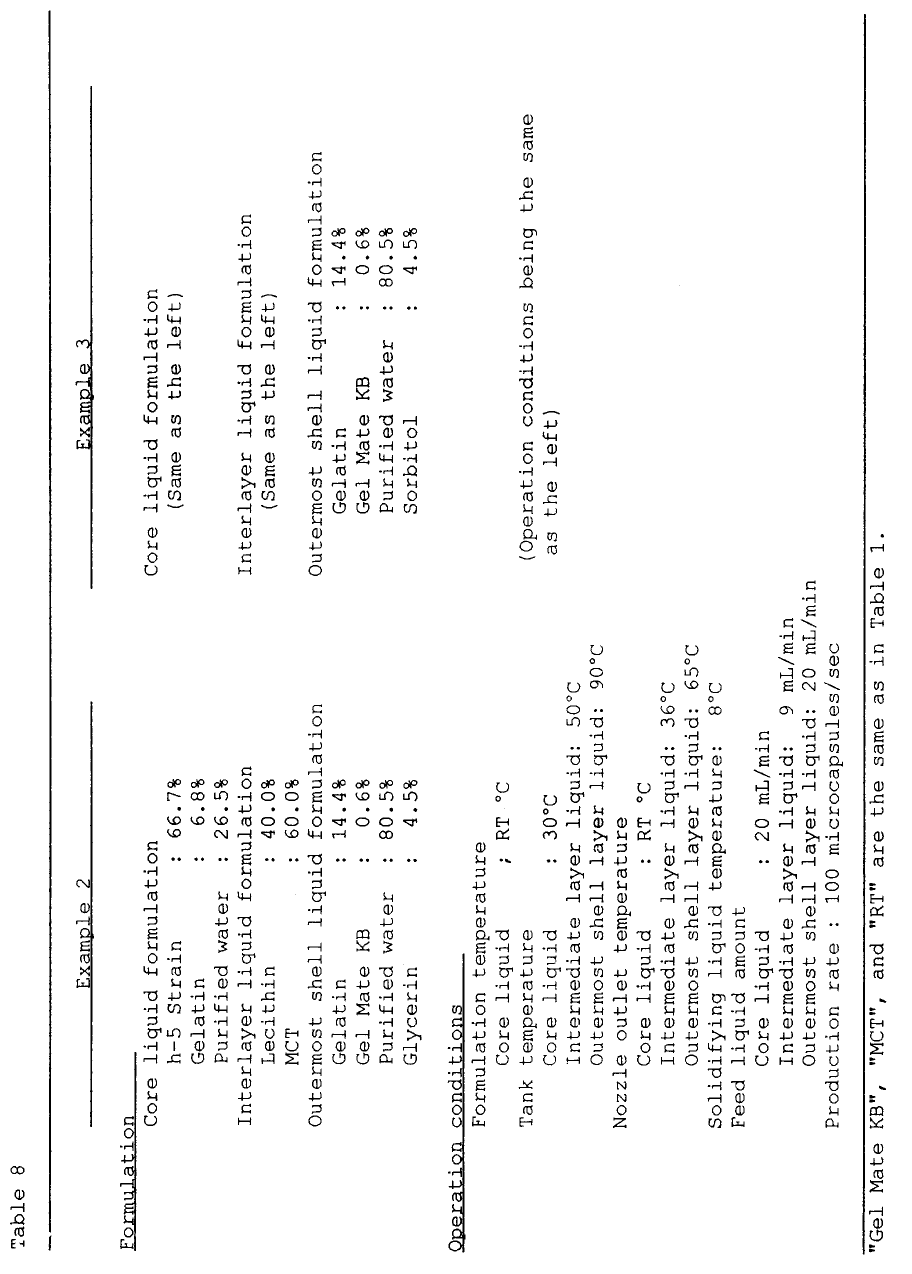

**Table 9**

| Example 1 | | Example 2 | |
|---|---|---|---|
| | | | |

| Microcapsules | | | |
|---|---|---|---|
| Before drying | | Before drying | |
| Particle diameter | 3.0 mm⌀ | Particle diameter | 3.0 mm⌀ |
| Weight | 14.1 mg | Weight | 14.1 mg |

| After drying | | After drying | |
|---|---|---|---|
| Particle diameter | 2.1 mm⌀ | Particle diameter | 2.1 mm⌀ |
| Weight | 4.6 mg | Weight | 4.6 mg |

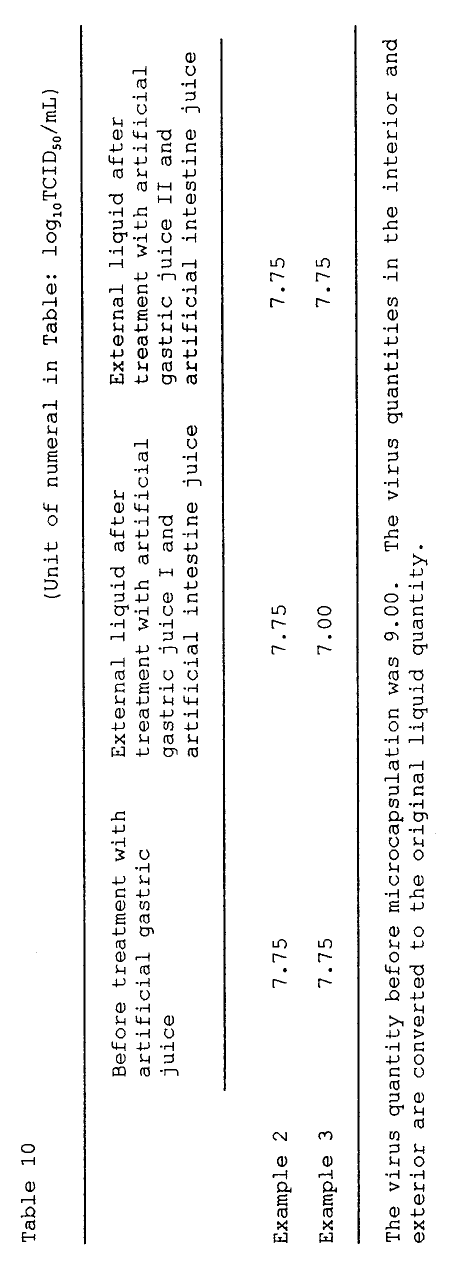

## Claims

1. An immunogen-containing mini-capsule for immunizing humans or animals, having a plural-layer structure comprising an immunogen-containing core layer, and one or more shell layers for covering the core layer, the outermost shell layer at least being composed of an intestine-soluble material having shape-retaining ability for retaining the spherical shell structure at room temperature.

2. The immunogen-containing mini-capsule according to claim 1, wherein the flow temperature of the outermost layer is not lower than 20°C.

3. The immunogen-containing mini-capsule according to claim 1 or 2, wherein the flow temperature of the intermediate layer is not lower than 10°C.

4. The immunogen-containing mini-capsule according to any of claims 1 to 3, wherein the intestine-soluble material is a mixture of gelatin with a gum or gums selected from the group of gellan gum, carrageenan, xanthane gum, and gum arabic.

5. The immunogen-containing mini-capsule according to any of claims 1 to 4, wherein the core layer containing the immunogen is an aqueous solution.

6. A process for producing the immunogen-containing mini-capsule set forth in any of claims 1 to 5, comprising extruding a liquid suspension of an immunogen from the center tube of a concentric multiple nozzle, and extruding simultaneously an aqueous solution of an intestine-soluble material from the outermost tube of the concentric multiple nozzle to allow the extruded matter to drop into a bath for solidification thereof.

7. The process for producing the immunogen-containing mini-capsule according to claim 6, wherein a concentric triple nozzle is employed for the production of the mini-capsule, an aqueous immunogen suspension is extruded from the center tube of the concentric triple nozzle, a hydrophobic material having a flow temperature of not lower than 10°C is extruded from the intermediate tube, and an aqueous solution of the intestine-soluble material is extruded from the outermost tube.

8. A method of immunizing an animal, comprising administering orally the immunogen-containing mini-capsules set forth in any of claims 1 to 5 to an animal.
